# EUROPEAN PATENT APPLICATION

(11) **EP 4 579 992 A1**
(43) Date of publication of application: **02.07.2025**
(21) Application number: 24222326.1
(22) Date of filing: 20.12.2024
(51) Int. Cl.: H02J 7/00, A61N 1/39

(54) **SYSTEMS, METHODS, AND APPARATUS FOR DETECTING THE END OF LIFE OF A BATTERY**

(30) Priority: 29.12.2023 US 202363616383 P
(71) Applicant: Physio-Control, Inc., Redmond, WA 98052 (US)
(72) Inventor: RUTZER, Mark, Redmond, 98052 (US); NORDNESS, Rocky, Redmond, 98052 (US); APPERSON, Ryan, Redmond, 98052 (US); EDMONSON, Kristina, Redmond, 98052 (US); BARTLETT, Chad, Redmond, 98052 (US)
(74) Representative: V.O.

(57) **Abstract**

A portable medical device is disclosed. The portable medical device may comprise a display, a defibrillator port, a battery unit, and a processor. The processor may be configured to determine a cycle count of the battery unit, wherein the cycle count represents a number of times the battery unit has been charged and determine whether the cycle count satisfies a cycle count threshold. The processor may also be configured to, in response to determining that the cycle count satisfies the cycle count threshold, cause one or more graphical elements to be displayed on the display.

## Description

### FIELD

The present disclosure relates generally to medical devices and, more particularly, to systems and methods for detecting the end of the life of a battery powering an electronic device, such as a portable medical device.

### BACKGROUND

There are a number of medical devices that utilize one or more battery packs or units to provide power thereto. Over time and/or repeated charge cycles, the health of the battery packs can deteriorate. Deteriorating health of the battery packs can reduce the effectiveness and performance of the medical devices, and potentially result in a failure.

Examples of medical devices where monitoring battery health is important include portable external defibrillators. There are several types of external defibrillators, including manual defibrillators, which are generally used by medical personnel, and automated external defibrillators, commonly known by the acronym AED, which are designed for use by laypersons.An AED is typically a small, portable device that analyzes the heart's rhythm and if the analysis determines that a defibrillating shock is advisable, either prompts the user to deliver a defibrillation shock or delivers a defibrillation shock without user interaction. Once a typical AED is activated, it can guide the user through each step of the defibrillation process by providing instructions in the form of aural or visual prompts.

Commercially available AED's may analyze the patient's heart rhythm and charge an energy storage device before a defibrillating shock can be delivered to a person or patient experiencing a medical condition, such as a cardiac arrest. This is done using a decision-making algorithm commonly referred to as a shock advisory algorithm. Current commercially available defibrillators may analyze an electrocardiogram (ECG) to determine if the person's heart is in a condition where delivery of a defibrillating shock is advisable, and after this analysis is done, charge if a shock is recommended.

The defibrillation shock provided by portable external defibrillators is a high-energy defibrillation pulse to the chest of a person to cause the person's heart to stop fibrillating and return to a normal rhythm. Sometimes the application of a single defibrillation pulse fails to restore the person's heart to a normal rhythm. In such an event, it may be necessary to apply additional defibrillation pulses. Further, portable external cardiac defibrillators are typically required to perform a range of monitoring tasks, including conducting ECG testing and monitoring compression and ventilations during cardiopulmonary resuscitation (CPR) treatment.
Portable external cardiac defibrillators are typically used by first responders or laypersons in the field, where alternative energy sources for powering the devices or recharging batteries may not readily available. Portable external cardiac defibrillators generally use a battery pack or unit to power the defibrillator. The battery pack may allow an energy storage device (e.g., a capacitor) to be charged in order to generate a defibrillation pulse. This operation can require drawing a high level of power from the battery pack over a short period of time. If the battery pack of a portable external cardiac defibrillator is at its end of life or becomes depleted, the defibrillator may not be able to be used to treat a person experiencing a cardiac arrest and the battery pack may need to be recharged or replaced. In order to assure that a portable external defibrillator is always ready for use, it is beneficial to monitor the battery health of the defibrillator and alert the user of the defibrillator before the battery pack is depleted or is at its end of life.

### SUMMARY

The present application is directed to embodiments relating to systems, methods, and apparatus for battery monitoring and/or management of a device, such as a medical device. The embodiments may be configured to monitor conditions and parameters of one or more battery units of the device. The conditions and parameters of the battery units may be evaluated to detect the end of life of one or more of the battery units. For example, the embodiments may monitor battery voltages, charging cycles, absolute state of charges (ASOC), battery currents, charging currents, charging voltages, discharge cycles, dates of battery first use, battery manufacturing dates, charge capacities, battery temperatures, and/or combinations thereof. The embodiments may provide an indication that one or more of the battery units is nearing or at the end of life. Further, the embodiments may select one or more of the battery units to provide power and/or may adjust the charging rate of an energy storage device based on the conditions and parameters of the one or more battery units of the device.

In one aspect, a portable medical device is disclosed. The portable medical device may comprise a display, a defibrillator port, a battery unit, and a processor. The processor may be configured to determine a cycle count of the battery unit. The cycle count represents a number of times the battery unit has been charged. The processor may also be configured to determine whether the cycle count satisfies a cycle count threshold and/or other battery condition thresholds are met. Further, the processor may be configured to, in response to determining that the cycle count satisfies the cycle count threshold, cause one or more graphical elements to be displayed on the display.

In another aspect, a method for monitoring a battery unit of a medical device is disclosed. The method may comprise determining a cycle count of the battery unit and/or other battery conditions. The method may also comprise determining whether the cycle count satisfies a cycle count threshold and/or other battery condition thresholds are met. Further, the method may comprise causing one or more graphical elements to be displayed in response to determining that the cycle count and/or other battery conditions satisfy a threshold.

In still another aspect, a non-transitory computer-readable medium storing instructions is disclosed that, when the instructions are executed by one or more processors, causes the one or more processors to perform operations for monitoring a battery unit of a medical device. The operations may comprise determining a cycle count of a battery unit of a medical device and/or other battery conditions. The operations may also comprise determining whether the cycle count satisfies a cycle count threshold and/or other battery condition thresholds are met. Further, the operations may comprise causing one or more graphical elements to be displayed in response to determining that the cycle count and/or other battery conditions satisfy a threshold.

In yet another aspect, a portable medical device is disclosed. The portable medical device may comprise a defibrillator port, a battery unit, a charging circuit, an energy storage device, and a processor. The processor may be configured to determine a temperature and an absolute state of charge (ASOC) of a battery unit. The processor may also be configured to determine whether the temperature of the battery unit satisfies a temperature threshold and determine whether the ASOC of the battery unit satisfies a ASOC threshold. Further, the processor may also be configured to, in response to determining that the temperature satisfies the temperature threshold and the ASOC satisfies the ASOC threshold, cause a charging circuit to charge an energy storage device at a first charging rate.

In another aspect, a method for charging an energy storage device of a portable medical device is disclosed. The method may comprise charging the energy storage device of the portable medical device at a first charging rate and determining a temperature and an absolute state of charge (ASOC) of the battery unit. The method may also be comprises determining whether the temperature of the battery unit satisfies a temperature threshold and determining whether the ASOC of the battery unit satisfies an ASOC threshold. Further, the method may also comprise, in response to determining that the temperature satisfies the temperature threshold and the ASOC satisfies the ASOC threshold, cause the charging circuit to charge the energy storage device at a second charging rate.

In another aspect, a portable medical device is disclosed. The portable medical device may comprise a defibrillator port, a battery unit, a charging circuit, an energy storage device, and a processor. The processor may be configured to determine a voltage level of the battery unit during charging of the energy storage device. The processor may also be configured determine whether the voltage level of the battery unit satisfies a voltage threshold. Further, the processor may also be configured to, in response to determining that the voltage level satisfies the voltage threshold, cause the charging circuit to charge the energy storage device at a second charging rate.

In another aspect, a method for charging an energy storage device of a portable medical device is disclosed. The method may comprise charging the energy storage device at a first charging rate. The method may also comprise determining a voltage level of a battery unit of the portable medical device during charging the energy storage device and determining whether the voltage level of a battery unit satisfies a voltage level threshold. Further, the method may also comprise, in response to determining that the voltage level satisfies the voltage level threshold, adjusting the first charging rate to a second charging rate.

The foregoing summary is illustrative only and is not intended to be in any way limiting. In addition to the illustrative aspects, embodiments, and features described above, further aspects, embodiments, and features will become apparent by reference to the figures and the following detailed description.

### BRIEF DESCRIPTION OF THE DRAWINGS

A more complete understanding of embodiments of the present application may be derived by referring to the detailed description and claims when considered in conjunction with the following figures, wherein like reference numbers may refer to similar elements throughout the figures. The figures are provided to facilitate understanding of the disclosure without limiting the breadth, scope, scale, or applicability of the disclosure. The drawings are not necessarily made to scale.
FIG. 1 is a diagram illustrating a representation of an example scene showing the use of a medical device for monitoring and delivering treatment to a person or patient experiencing a medical condition, in accordance with an example implementation;
FIG. 2 illustrates a front view of a medical device, in accordance with an example implementation;
FIG. 3 illustrates a simplified block diagram of the components of a medical device, in accordance with an example implementation;
FIG. 4 illustrates a graphical user interface of a medical device, in accordance with an example implementation;
FIG. 5 is a table illustrating battery parameters for selecting one or more of battery units to provide power for a medical device, in accordance with an example implementation;
FIG. 6 is a table illustrating battery parameters for selecting charging rates for a battery unit of a medical device, in accordance with an example implementation;
FIG. 7 is a table illustrating battery parameters for selecting charging rates for battery units of a medical device, in accordance with an example implementation;
FIG. 8 is a flowchart illustrating a method of detecting the end of life of a battery unit of a medical device, in accordance with an example implementation;
FIG. 9 is a flowchart illustrating a method for charging an energy storage device of a medical device, in accordance with an example implementation; and
FIG. 10 is a flowchart illustrating a method for charging an energy storage device of a medical device, in accordance with another example implementation.

### DETAILED DESCRIPTION

The figures and the following description illustrate specific exemplary embodiments. It will be appreciated that those skilled in the art will be able to devise various arrangements that, although not explicitly described or shown herein, embody the principles described herein and are included within the scope of the claims that follow this description. Furthermore, any examples described herein are intended to aid in understanding the principles of the disclosure and are to be construed as being without limitation. As a result, this disclosure is not limited to the specific embodiments or examples described below, but by the claims and their equivalents.

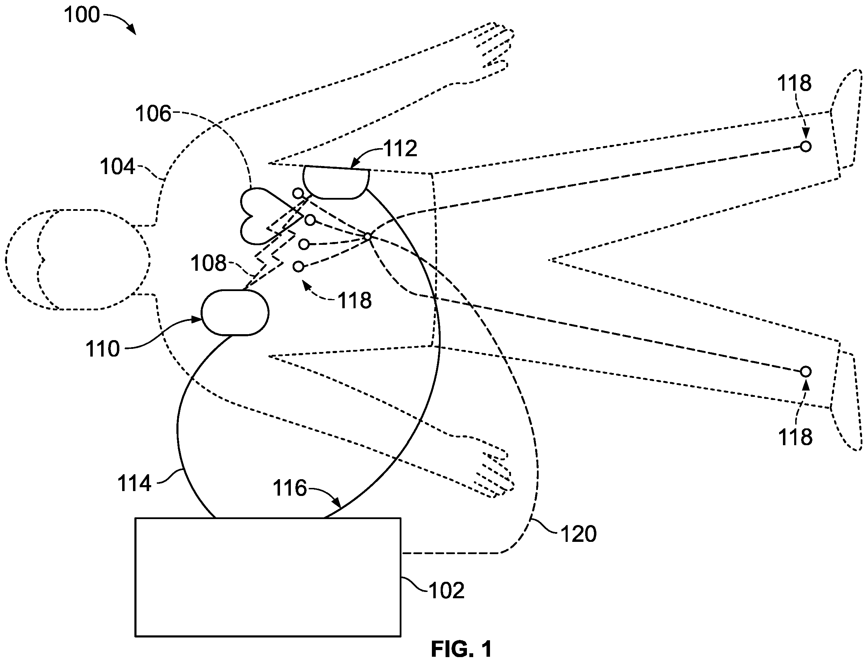

Particular embodiments are described herein with reference to the drawings. In the description, common features may be designated by common reference numbers throughout the drawings. In some drawings, multiple instances of a particular type of feature may be used. Although these features are physically and/or logically distinct, the same reference number is used for each, and the different instances are distinguished by addition of a letter to the reference number. When the features as a group or a type are referred to herein (e.g., when no particular one of the features is being referenced), the reference number is used without a distinguishing letter. However, when one particular feature of multiple features of the same type is referred to herein, the reference number is used with the distinguishing letter. For example, referring to Figure 1, sensors are illustrated and associated with reference number 118. When referring to a particular one of the sensors, such as the sensor 118A, the distinguishing letter "A" may be used. However, when referring to any arbitrary one of the sensor or to the sensors as a group, the reference number 118 may be used without a distinguishing letter.

As used herein, various terminology is used for the purpose of describing particular implementations only and is not intended to be limiting. For example, the singular forms "a," "an," and "the" are intended to include the plural forms as well, unless the context clearly indicates otherwise. Further, the terms "comprise," "comprises," and "comprising" are used interchangeably with "include," "includes," or "including." Additionally, the term "wherein" is used interchangeably with the term "where." As used herein, "exemplary" indicates an example, an implementation, and/or an aspect, and should not be construed as limiting or as indicating a preference or a preferred implementation. As used herein, an ordinal term (e.g., "first," "second," "third," etc.) used to modify an element, such as a structure, a component, an operation, etc., does not by itself indicate any priority or order of the element with respect to another element, but rather merely distinguishes the element from another element having a same name (but for use of the ordinal term). As used herein, the term "set" refers to a grouping of one or more elements, and the term "plurality" refers to multiple elements.

The present application is directed to embodiments relating to systems, methods, and apparatus for battery monitoring and/or management of a device, such as a medical device. The embodiments may be configured to monitor conditions or parameters of one or more battery units of the device. The conditions and parameters of the battery units may be evaluated to detect an end of life condition of the one or more battery units. For example, the embodiments may monitor battery voltages, charging cycle, absolute state of charges (ASOC), battery currents, charging currents, charging voltages, discharge cycles, dates of battery first use, battery manufacturing dates, charge capacities, battery temperatures, and/or combinations thereof. The embodiments may provide an indication to a user that one or more of the battery units is nearing or at the end of life. Further, the embodiments may select one or more of the battery units to provide power and/or may adjust the charging rate of an energy storage device based on the conditions and parameters of the one or more battery units.

Referring now to the figures, Figure 1 is a diagram of a representation of an exemplary scene 100 showing use of a medical device 102 for monitoring and providing treatment or therapy to a person 104 experiencing a medical condition, such as a cardiac arrest. The medical device 102 may be operated by a user (e.g., a healthcare professional, service worker, a doctor, a first responder, etc.) and may be used in a hospital or a pre-hospital environment or setting. The medical device 102 may include functions and operations of an external defibrillator, such as a manual defibrillator, an automatic external defibrillator (AED), or any other suitable defibrillator. In some examples, the medical device 102 may be a monitor defibrillator, which is a combination of a monitor and a defibrillator.

As shown in Figure 1, the medical device 102 is positioned near the person 104 (e.g. patient). The person 104 may be experiencing a condition in his or her heart 106, which could be, for example, cardiac arrest or any other cardiac rhythm abnormality. The person 104 may be lying on his or her back on a surface, such as the ground or a bed, and may be located in a hospital, a home, or a pre-hospital environment (e.g., an ambulance). The medical device 102 may be configured to generate an electrical pulse 108 and deliver the electrical pulse 108 to the person 104. The electrical pulse 108, also known as a defibrillation shock or therapy shock, is intended to go through and restart the heart 106, in an effort to save the life of the person 104. The electrical pulse 108 can further include one or more pacing pulses and the like.

The electrical pulse 108 may be delivered to the person 104 using defibrillation electrodes 110 and 112. The defibrillation electrodes 110 and 112 may include hand-held electrode paddles or electrode pads placed on the body of the person 104. An electrical cable 114 may connect the defibrillation electrode 110 to the medical device 102 and an electrical cable 116 may connection the defibrillation electrode 112 to the medical device 102. When the defibrillation electrodes 110 and 112 are in electrical contact with the body of the person 104, the medical device 102 may administer, via the defibrillation electrodes 110 and 112, the electric pulse 108 through the heart 106 of person 104. The defibrillation electrodes 110 and 112 may also be configured to sense or detect one or more conditions and/or physiological parameters of the person 104 and generate signals representative of the conditions and physiological parameters.

As shown in phantom in FIG. 1, one or more sensors or electrodes 118 may be placed at various locations on the body of the person 104. The sensors 118 may be configured to sense or detect conditions and/or physiological parameters of the person 104 and produce signals representative of the conditions and physiological parameters. An electrical cable 120 may connect the sensors 118 to the medical device 102. The physiological parameters generated by the sensors 118 and/or the defibrillation electrodes 110 and 112 may be provided to the medical device 102 for analysis. The physiological parameters may include ECG data, heart rhythm data, heart rate data, cardiac output data, blood flow data, a level of perfusion, respiration data, body temperature data, and/or any other suitable physiological parameter that may be used to assess the physical condition of the person 104.

The medical device 102 may be configured to select an appropriate treatment protocol based on the physiological parameters. For example, the medical device 102 may determine a cardiopulmonary resuscitation (CPR) treatment to apply to the person 104. The medical device 102 may also determine whether a defibrillation pulse should be applied or delivered to the person 104. Further, the medical device 102 may display the representations of the conditions and/or physiological parameters of the person 104 to assist a user in treating and diagnosing medical conditions.

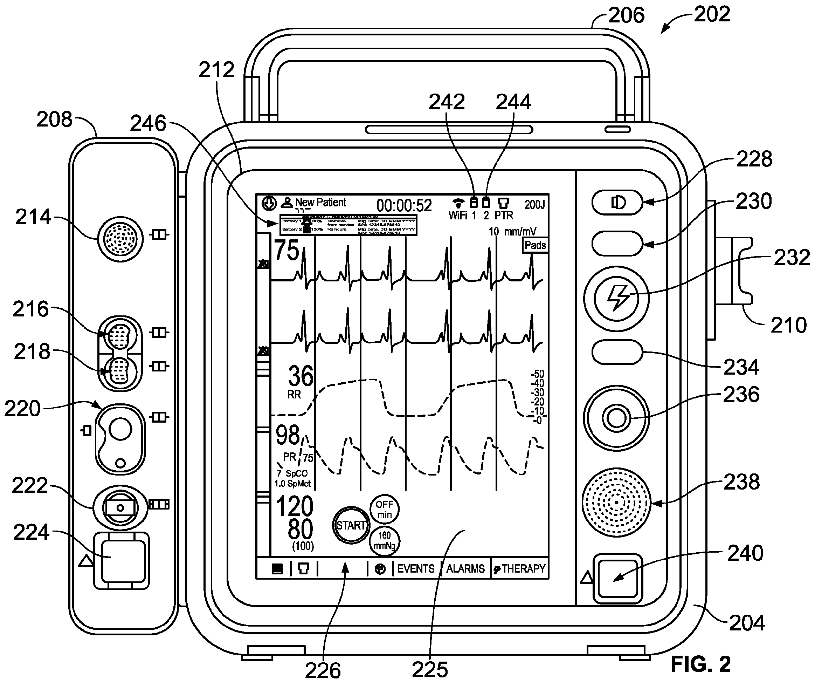

Figure 2 illustrates a front view of a medical device 202, in accordance with an example implementation. The medical device 202 can comprise the medical device 102 of Figure 1. The medical device 202 may be configured to monitor and provide treatment or care to a person or patient experiencing a medical condition, such as a cardiac arrest or any other cardiac rhythm abnormality. The medical device 202 may be operated by a user (e.g., a medical professional, a first responder, a healthcare professional, service worker, a doctor, etc.) and may be used in a hospital or a pre-hospital environment or setting. As illustrated, the medical device 202 may be a monitor defibrillator, which is a combination of a monitor and a defibrillator.

As a defibrillator, the medical device 202 may be configured to deliver an electrical pulse or shock to a person experiencing a medical condition. The medical device 202 may be configured to operate or function in one or more defibrillation modes, such as a manual defibrillation mode, an AED mode, or any other suitable defibrillation mode. For example, the medical device 202 may be selected to operate in an AED mode when the medical device 202 is intended to be used by first responders and/or people who are not trained in providing medical treatment using defibrillation. When operating in the AED mode, the medical device 202 may determine whether a defibrillation pulse or shock is needed and, if so, charge an energy storage device (e.g., a capacitor) of the medical device 202 to a predetermined energy level and instruct the user to administer the defibrillation shock. The medical device 202 may also be configured to operate in a manual defibrillation mode when the medical device 202 is intended to be used by persons who are trained in providing medical treatment using defibrillation (e.g., medical professionals, such as doctors, nurses, paramedics, emergency medical technicians, etc.). When the medical device 202 is configured to operate in the manual defibrillation mode, the device may provide an array of vital signs of a person to the user, including ECG data (as discussed below in connection with Figure 2), allowing user interpretation of the vital signs, and allows the user to change the selected energy (although configuration options may set a default energy), charge the defibrillator energy storage device, and deliver a shock to the person.

As a monitor, the medical device 202 can monitor and evaluate conditions and physiological parameters of a person being treated for a medical condition. The medical device 202 may receive or acquire signals or voltages from one or more electrodes or sensors placed at various locations on the body of the person. The electrodes may sense or detect the physiological parameters of the person and produce signals representative of the physiological parameters. The representations of the physiological parameters may be displayed by the medical device 202 to assist a user in treating and diagnosing medical conditions of the person. The physiological parameters may include ECG (electrocardiogram) data, body temperature, non-invasive blood pressure (NIBP), arterial oxygen saturation/pulse oximetry (SpO2), a concentration or partial pressure of carbon dioxide in the respiratory gases (e.g., capnography), and/or any other suitable physiological parameter of a person. These physiological parameters may be stored in the memory of the medical device 202. In some examples, the physiological parameters may be transmitted to other remote communication or computing devices and databases.

As shown in Figure 2, the medical device 202 includes a housing or casing 204, a handle 206, an input module or interface 208, a defibrillation interface 210, and a user interface 212. The handle 206 of the medical device 202 may be attached to the housing 204 to allow a user to move or transport the medical device 202 to a location near a person experiencing a medical condition. The housing 204 may be generally rectangular or square shaped. In other examples, the housing 204 may have any other suitable shape. The housing 204 may be formed from various materials or combinations of materials. For example, the housing 204 may be made of molded plastic, metal, or some combination of both.

The input module 208 of the medical device 202 may be coupled to or integral with the housing 204. The input module 208 may enable the medical device 202 to receive vital signs and other physiological parameters (e.g., a heart rate (HR) and ECG data) of a person via sensors (e.g., the sensors 118 of Figure 1). The sensors may be placed on the body of a person to sense or detect signals generated by the body or heart of the person. The input module 208 may include one or more ports or receptacles to enable the sensors to be detachably coupled to the input module 208. As shown in Figure 2, the input module 208 can include a port 214 configured to be connected to an oxygen saturation (SpO2) sensor, a port 216 configured to be connected to a temperature sensor, a port 218 configured to be connected to a sensor for measuring invasive blood pressure (IP) via a catheter, a port 220 configured to be connected to a sensor for measuring partial pressure of carbon dioxide (CO2) in gases in the airway via capnography, and a port 222 configured to be connected to sensor for measuring a non-invasive blood pressure (NIBP). The input module 208 may also include a communication port 224, such as a Universal Serial Bus (USB) port, that can be used to connect to an input device (e.g., a mouse, a keyboard, etc.). The input module 208 may include other ports to enable any other suitable physiologic parameter of a person to be monitored and evaluated by the medical device 202.

The defibrillation interface 210 of the medical device 202 may be configured to enable electrical charges or pulses to be delivered or applied to a person via defibrillation electrodes (e.g., the defibrillation electrodes 110 and 112 of Figure 1). The defibrillation electrodes may also be configured to enable the medical device 202 to receive physiological parameters of a person (e.g., a heart rate (HR), ECG data, etc.). The defibrillation interface 210 may include one or more ports or receptacles capable of allowing defibrillation electrodes to be detachably coupled to the defibrillation interface 210. A cable assembly or therapy cable may enable the defibrillation electrodes to be coupled to the ports of the defibrillation interface 210. Each therapy cable may include a defibrillation electrode attached at one end and a connector attached to the other end. The connector may be configured to be coupled to or plugged into a port or receptacle of the defibrillation interface 210.

The user interface 212 of the medical device 202 may include one or more input devices configured to receive inputs or commands from a user and include one or more output devices configured to provide information to the user. The input devices of the user interface may include various controls, such as switches, pushbuttons, keyboards, touchscreens, microphones, scanners, and/or cameras, etc., for operating the medical device 202. The output devices of the user interface 212 can be visual, audible or tactile, for communicating to a user of the medical device 202 (e.g., a medical professional, a first responder, etc.). For example, the output devices may be configured to present visual alarms or alerts, flashing lights, and/or warnings to the user. The output devices may also include an audio system that provides audio signals to aurally communicate with the user voice prompts that deliver instructions or commands, monotonal, ascending, descending or quickening tones to indicate alerts or warnings, or any other suitable audio signals for communicating with the user of the medical device 202.

As shown in Figure 2, the user interface 212 of the medical device 202 includes a power button 228 configured to turn the medical device on and off (e.g., "On-Off" button), a charge button 230 configured to cause the medical device 202 to build an electric charge to be applied to the person in a form of a defibrillation shock or pulse, a defibrillation shock button 232 configured to cause the medical device 202 to apply a defibrillation pulse or therapy shock to a person during a defibrillation episode, an analyze button 234 configured to cause the medical device 202 to analyze physiologic parameters of a person (e.g., ECG data) to facilitate determining the appropriate time to apply a defibrillation pulse or shock, and a speed dial button 236 configured to navigate through menus of on-screen software. The user interface 212 may also include a speaker 238 to provide audio output and a USB output port 240 to facilitate connecting the medical device 202 to a device such as a printer. The user interface 212 may include any other suitable output device for providing outputs or input device for receiving inputs from a user.

The user interface 212 of the medical device 202 may also include a display device 225 (e.g., a touchscreen) for displaying a graphical user interface (GUI) 226. The GUI 226 can display conditions and/or physiologic parameters of a person (e.g., a patient), provide visual feedback to the user (e.g., a healthcare provider) about a condition of the person, provide information about the medical device 202 (e.g., battery information), and allow the user to interact with and operate the medical device 202. The GUI 226 may also be configured to visually present various measured or calculated parameters associated with the person indicating the physical status of the person (e.g., an ECG), and/or instructions and/or commands, including prompts to perform cardiopulmonary resuscitation (CPR) treatment or other treatment instructions, to the user. Further, the GUI 226 may include multiple visual user interface items that are selectable or "clickable" by the user including user-selectable icons, user-selectable on-screen buttons, menus, widgets, scroll bars, graphical objects, and other items for facilitating user interaction.

As shown in Fig 2, the GUI 226 includes graphical representations of a first battery unit indicator 242 and a second battery unit indicator 244. The first battery unit indicator 242 and second battery unit indicator 244 may be configured to display a status of a first battery unit and a status of a second battery unit, respectively. For example, the first and second battery unit indicators 242 and 244 may provide a charging level of the first and second battery units. The GUI 226 may also display messages and information about end of life conditions of the first battery unit and/or the second battery unit. As shown, the GUI 226 may indicate that the first battery unit should be removed from service. The GUI 226 may also provide additional information about the battery units of the medical device 202, such as battery charge levels (e.g., a remaining charge), manufacturing dates, serial numbers, etc. Further, the GUI 226 may provide audible alarms or warnings when a battery unit of the medical device 202 has reached its end of life and/or should be removed and replaced.

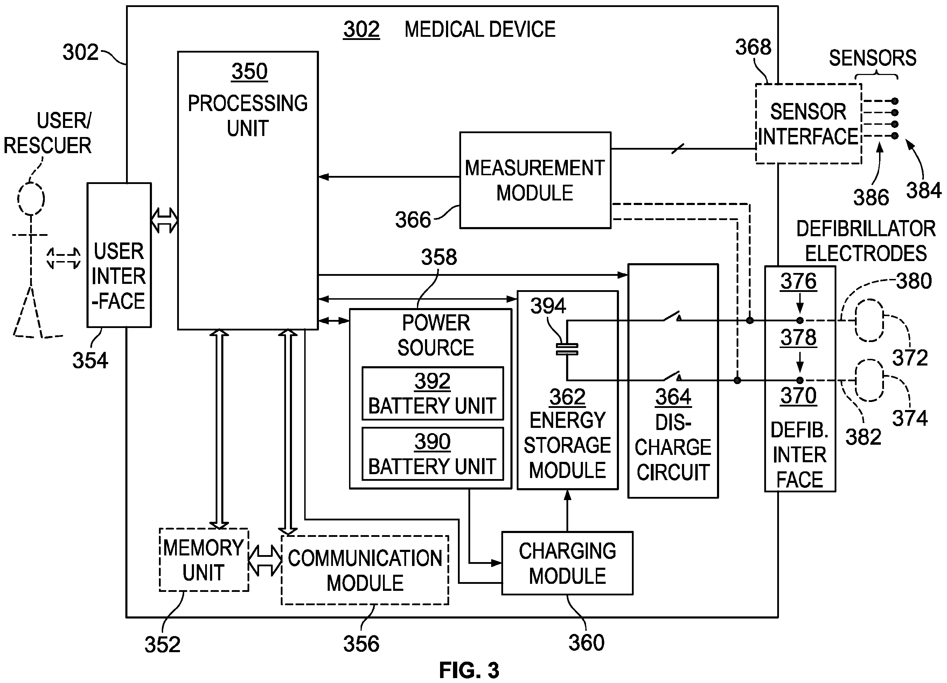

FIG. 3 is a diagram showing components of a medical device 302, in accordance with an exemplary embodiment. As shown in Figure 3, the medical device 302 includes a processing unit 350, a memory unit 352, a user interface 354, a communication module or interface 356, a power source or module 358, a charging module 360, an energy storage module or device 362, a discharge circuit 364, a measurement module 366, a sensor interface 368, and a defibrillator interface 370. These components can be, for example, included in the medical devices of Figure 1 or 2.

The defibrillation interface 370 of the medical device 302 may be configured to enable an electrical pulse or shock to be delivered or applied to a person (e.g., a patient) experiencing a medical condition. The defibrillation interface 370 may comprise the defibrillation interface 210 of Figure 2. The defibrillation interface 370 may include one or more ports or nodes 376 and 378 to enable the defibrillation electrodes 372 and 374 to be detachably coupled to the defibrillation interface 370. A cable assembly or therapy cable 380 may enable a defibrillation electrode (e.g., defibrillation electrode 372) to be coupled to the port 376 of the defibrillation interface 370 and a cable assembly or therapy cable 382 may enable a defibrillation electrode (e.g., defibrillation electrode 374) to be coupled to the port 378 of the defibrillation interface 370 of the medical device 302. The cable assemblies 380 and 382 may each include a connector configured to be coupled to or plugged into the ports 376 and 378 of the defibrillation interface 370. The connectors may be male or female connectors that are compatible with the ports 376 and 378 of the defibrillation interface 370 of the medical device 302.

The defibrillation interface 370 may also enable the medical device 302 to receive physiological parameters (e.g., a heart rate (HR) and ECG data) of the person from the defibrillation electrodes 372 and 374. Each of the defibrillation electrodes 372 and 374 may be configured to measure one or more physiological parameters of the person (e.g., heart electrical activity, heart rate, etc.) and to provide signals representative of the physiological parameters to the defibrillation interface 370. For example, when the defibrillation electrodes 372 and 374 are placed on the chest of the person, an ECG signal of the person can be detected as a voltage difference between the defibrillation electrodes 372 and 374. The defibrillation electrodes 372 and 374 may also be used to determine device parameters indicative of a condition of the medical device, such as an electrode impedance or a user interaction with the medical device 302. For example, the medical device 302 can detect an impedance between the defibrillation electrodes 372 and 374 to determine whether the defibrillation electrodes 372 and 374 are making sufficient electrical contact with a person's body.

The sensor interface 368 of the medical device 302 may also be configured to receive physiological parameters (e.g., a heart rate (HR), ECG data, etc.) from one or more sensors 384 (e.g., physiological sensors). The sensors 384 may be placed in contact with the body of a person being monitored or treated for a medical condition. Each of the sensors 384 may include a transducer configured to sense a signal or voltage of the person. For example, the sensors 384 may measure or detect heart electrical activity, such as an electrocardiogram (ECG), saturation of the hemoglobin in arterial blood with oxygen (SpO2), carbon monoxide (carboxyhemoglobin, COHb) and/or methemoglobin (SpMet), partial pressure of carbon dioxide (CO2) in gases in the airway by means of capnography, total air pressure in the airway, flow rate or volume of air moving in and out of the airway, blood flow, blood pressure (e.g., non-invasive blood pressure (NIBP)), core body temperature with a temperature probe in the esophagus, oxygenation of hemoglobin within a volume of tissue (rSO2), and any other physiological parameters of a person being monitored or treated.

The sensor interface 368 may include one or more receptacles or ports (e.g., an ECG port) to enable the sensors 384 to be detachably coupled to the medical device 302. The sensors 384 may be attached to the sensor interface 368 by cable assembles or therapy cables 386. In some embodiments, the sensors 384 may be fixedly connected to the sensor interface 368. The therapy cables 386 may each include a sensor 384 at one end and to a connector at the opposite end. The connector can be configured to be coupled to or plugged into a port or receptacle of the sensor interface 368.

The measurement module 366 of the medical device 302 may be configured to receive signals or sensor data from the sensor interface 368 and the defibrillation interface 370. The signals may be representative of the conditions and/or physiological parameters of a person being monitored and/or treated for a medical condition. The measurement module 366 may measure or determine various conditions and physiological parameters from the signals. For example, the measurement module 366 may determine an ECG of the person based on a voltage difference between the defibrillation electrodes 372 and 374. The measurement module 366 may also determine device parameters indicative of a condition of the medical device 302, such as an electrode impedance or a user interaction with the medical device 302. For example, the measurement module 366 may measure an impedance or voltage across the defibrillation electrodes 372 and 374 of a pair of sensor.

The measurement module 366 may also include a filter circuit or hardware (e.g., amplifiers, filters, etc.) to attenuate and/or filter at least some of the noise that may be present on the signals received from the sensor interface 368 and/or the defibrillation interface 370. For example, the filter circuit may apply at least one filter to the signal to remove artifacts resulting from chest compressions being delivered to the person. In some embodiments, the filter may be implemented as an analog filter, a digital filter, or combinations of both. The measurement module 366 may also digitize the signals received from the sensor interface 368 and/or defibrillation interface 370 prior to transmitting the signals to the processing unit 350.

The memory unit 352 of the medical device 302 may be in operable communication with the processing unit 350. The memory unit 352 may store various values, look-up tables, equations, audio and video files, and/or plurality of treatment protocols that can be read and accessed by the processing unit 350. The treatment protocols may include instructions regarding CPR treatment (e.g., chest compressions). The memory unit 352 can also store a person's sensed physiological parameters, historical data, lengths of time and rate of CPR treatments, and defibrillation pulses previously discharged. Further, the memory unit 352 can store instructions or computer executable code of software routines that can be retrieved and executed by the processing unit 350.

The memory unit 352 may include one or more computer-readable storage media that can be read or accessed by the processing unit 350. The computer-readable storage media can include volatile and/or non-volatile memory, dynamic random-access memory (DRAM), read-only memory (ROM), random access memory (RAM), magnetic disk storage media, optical storage media, smart cards, flash memory devices, or any other suitable memory. In some embodiments, the computer-readable storage media can be integrated in whole or in part with the processing unit 350. The computer-readable storage media may be implemented using a single physical device (e.g., one optical, magnetic, organic or other memory or disc storage unit), while in other examples, the computer readable storage media can be implemented using two or more physical devices.

The processing unit 350 of the medical device 302 may be configured to control various operations of the medical device 302. The processing unit 350 may receive inputs from various components of the medical device 302 and process the inputs to produce outputs that may be stored in the memory unit 352 and/or displayed on the user interface 354. For example, the processing unit 350 may receive and evaluate conditions and physiological parameters of a person (e.g., electrical activity of the heart) from the sensors 384 and/or the defibrillation electrodes 372 and 374 placed on a person. The processing unit 350 may also determine whether a defibrillation pulse should be delivered to a person based upon the conditions and physiological parameters. For example, the processing unit 350 may make a shock/no shock determination based on ECG data or other information. In some examples, the processing unit 350 may cause the medical device 302 to automatically deliver defibrillation pulses in an AED mode or may advise the user of these determinations via the user interface 354. When a defibrillation shock has previously been delivered, the processing unit 350 may evaluate the efficacy of the delivered defibrillation pulse by determining if the heart of the person is still fibrillating based on the sensed electrical activity in order to determine whether an additional defibrillation pulse is warranted.

The processing unit 350 may also determine whether to commence charging of the energy storage module 362 of the medical device 302. In some embodiments, the processing unit 350 may determine the rate to charge the energy storage module 362. Further, in manual mode, the processing unit may cause an electrocardiogram (ECG) to be displayed on the user interface 354 that reflects the sensed electrical activity of a heart of a person. In addition, the processing unit 350 may control delivery of other types of treatment therapy to the person via the defibrillation electrodes 372 and 374, such as cardioversion or pacing therapy.

The processing unit 350 may include one or more general-purpose processors, special purpose processors (e.g., digital signal processors (DSP), application specific integrated circuits (ASIC), graphic processing units, etc.), or any other suitable processing unit or controller. In some implementations, the processing unit 350 may include a power processing unit and a therapy processing unit. The power processing unit may be configured to control the charging of the energy storage module 362 and the therapy processing unit may be configured to control the defibrillation interface 370. Further, the processing unit 350 may be configured to execute instructions (e.g., computer-readable program instructions) that are stored in memory and may be executable to provide the functionality of the medical device described herein. For example, the processing unit 350 can execute instructions for causing the medical device to display an ECG waveform on the user interface 354 of the medical device 302.

The user interface 354 of the medical device 302 may facilitate user interactions with the medical device 302. The user interface 354 may comprise the GUI 226 of Figure 2. The user interface 354 may include various types of input devices for receiving inputs or commands from a user. For example, the input devices may include keyboards, switches, microphones, pushbuttons, touchscreens, scanners, and/or any other suitable input device for enabling inputs to the medical device 302. For instance, a user may use the interface items displayed on the GUI to input information regarding a particular event (e.g., a treatment or medication administered to the person).

The user interface 354 may also comprise various types of output devices for providing information to the user. The output devices of the user interface 354 can be visual, audible or tactile for communicating to or providing feedback to a user (e.g. a rescuer, a first responder, a healthcare professional, etc.). The output devices may include a screen, one or more light emitting diodes (LEDs), and/or speakers to output various sounds (e.g., voice or audio), etc. For example, the output device can be configured to present visual alarms or alerts, flashing lights, and/or warnings to the user of the medical device. The output device may also include an audio system that provides an audio signal to aurally communicate with user voice prompts that deliver instructions or commands, monotonal, ascending, descending or quickening tones to indicate alerts or warnings, or any other suitable output device for communicating with the user.

The communication module 356 of the medical device 302 may be in communication with the processing unit 350. The communication module 356 may enable patient data, treatment information, CPR performance, system data, environmental data, etc. to be communicated between the medical device 302 and other devices, such as a remote assistance center and/or any other remote computing device. The communication module 356 may include one or more wireless or wireline interfaces that allow for both short-range communication and long range communication to one or more networks or to one or more remote devices. The wireless interfaces may provide for communication under one or more wireless communication protocols, such as Bluetooth, Wi-Fi (e.g., an institute of electrical and electronic engineers (IEEE) 802.xx protocol), Long-Term Evolution (LTE), cellular communications, near-field communication (NFC), radio-frequency identification (RFID), and/or other wireless communication protocols. Such wireline interfaces may include an Ethernet interface, USB interface, or similar interface to communicate via a wire, a twisted pair of wires, a coaxial cable, an optical link, a fiber-optic link, or other physical connection to a wireline network.

The power source or module 358 of the medical device 302 may provide power to the components of the medical device 302. The power source 358 may include one or more battery units. The battery units may include lithium batteries, alkaline batteries, nickel cadmium batteries, nickel metal hydride batteries, or any other suitable type of battery or energy device. As shown in Figure 3, the medical device 302 includes a first battery unit 390 and a second battery unit 392. The first battery unit 390 and second battery unit 392 are selectively coupled to the energy storage module 362 by the charging module 360.

The first battery unit 390 and second battery unit 392 may be rechargeable. In some examples, the power source 358 may include a charging cable or port for receiving a charging cable such that the power source 358 can be electrically coupled to an external power source. The external power source may be configured to charge the first battery unit 390 and/or second battery unit 392 while the first and second battery units 390 and 392 are coupled to the medical device 302. Alternatively or additionally, the first battery unit 390 and second battery unit 392 are each detachably coupled to the medical device 302 to enable a user to replace a defective or low charged battery unit with an additional battery unit. In some examples, after detaching the battery units, the first battery unit 390 and/or the second battery unit 392 can be coupled to a battery charger for recharging.

The first battery unit 390 and the second battery unit 392 may be continuously monitored by the processing unit 350. In some implementations, the processing unit 350 may monitor and evaluate the parameters and/or conditions of the first and second battery units 390 and 392 to determine the remaining battery charge and the end of life status for each of the first and second battery units 390 and 392. The remaining battery charge and the end of life status of the battery units 390 and 392 may be displayed to the user by indicators and/or messages displayed on a GUI of the medical device 302. For example, as shown in Figure 2, the first and second battery unit indicators 242 and 244 can indicate a present charge (e.g., remaining charge) of the first and second battery units 390 and 392 relative to the maximum battery charge. Further, the medical device 302 may provide end-of-battery life status or conditions to a user when the first battery unit 390 and/or the second battery unit 392 reaches its end of life and needs to be replaced. For example, when the first battery unit 390 and/or the second battery unit 392 has a remaining useful life below an end of life threshold, the processing unit 350 may indicate, via an end-of-battery life indicator, to a user to replace the first and/or second battery units 390 and 392.

The end-of-life status of the first and second battery units 390 and 392 may be determined based on one or more parameters and/or conditions of the first battery unit 390 and/or the second battery unit 392. For example, the processing unit 350 may determine the end of life status of a battery unit by monitoring a battery voltage, an absolute state of charge (ASOC), a battery current, a charging current, a charging voltage, charging cycles, a discharge cycle, a date of battery first use, a battery manufacturing date (e.g., age of battery), a charge capacity, a battery temperature, and/or combinations thereof. The monitored parameters and conditions of the first and second battery units 390 and 392 may be compared to each other and/or to predetermined threshold values to determine the end of life status of a battery unit. The operation of the medical device 302 may be adjusted by the processing unit 350 based on the end of life status of the first battery unit and/or the second battery unit 390 and 392. For example, when the a battery unit is near or at its end of life, the medical device 302 may reduce the amount of power being used from the battery unit or cease using power from the battery unit and only use power from the other battery unit.

In some implementations, the processing unit 350 may determine the end of the life status of a battery unit by monitoring the number of charging cycles of a battery unit. For example, the processing unit 350 may compare a charging cycle count of the first battery unit 390 and a charging cycle count of the second battery unit 392 to a first charging cycle count threshold and a second charging cycle count threshold, respectively. The first charging cycle count threshold and the second charging cycle count threshold may each represent a number of times a battery unit has been charged. The first charging cycle count threshold and a second charging cycle count threshold may be equal. For example, the first and second charging cycle count threshold may be about 300 charging cycles.

When the charging cycle count of the first battery unit 390 satisfies the first charging cycle count threshold (e.g., the charging cycle count of the first battery unit 390 is equal to and/or above the first charging cycle count threshold), the processing unit 350 may be configured to cause battery information to be provided or displayed indicating the first battery unit 390 is at its end-of-life and needs to be replaced. Similarly, when the charging cycle count of the second battery unit 392 satisfies the second charging cycle count threshold (e.g., the cycle count of the second battery unit 392 is equal to and/or above the second cycle count threshold), the processing unit 350 may be configured to cause battery information to be provided or displayed indicating the second battery unit 392 is at its end-of-life and needs to be replaced.

In some implementations, the processing unit 350 may determine the end of the life status of a battery unit based on the temperature of the battery unit and the absolute state of charge (ASOC). For example, the processing unit 350 may compare an ASOC of the first battery unit 390 and the ASOC of the second battery unit 392 to a first ASCO threshold and a second ASOC threshold, respectively. The first ASOC threshold and a second ASOC threshold may be equal. In some examples, the first and second ASOC threshold may be about 75 percent. Further, the processing unit 350 may compare the temperature of the first battery unit 390 and the temperature of the second battery unit 392 to a first temperature threshold and a second temperature threshold, respectively. The first temperature threshold and a second temperature threshold may be equal. In some examples, the first and second temperature threshold may be about 15 degrees Celsius.

When the ASOC of the first battery unit 390 satisfies the first ASOC threshold (e.g., the ASOC of the first battery unit 390 is equal to and/or above the first ASOC threshold) and the temperature of the first battery unit 390 satisfies the first temperature threshold (e.g., the temperature of the first battery unit 390 is equal to and/or above the first temperature threshold), the processing unit 350 may be configured to cause battery information to be provided or displayed indicating the first battery unit 390 is at its end of life and should be replaced. Similarly, when the ASOC of the second battery unit 392 satisfies the second ASOC threshold (e.g., the ASOC of the second battery unit 392 is equal to and/or above the second ASOC threshold) and the temperature of second battery unit 392 satisfies the second temperature threshold (e.g., the temperature of the second battery unit 392 is equal to and/or above the first temperature threshold), the processing unit 350 may be configured to cause battery information to be provided or displayed indicating the second battery unit 392 is at its end of life and should be replaced.

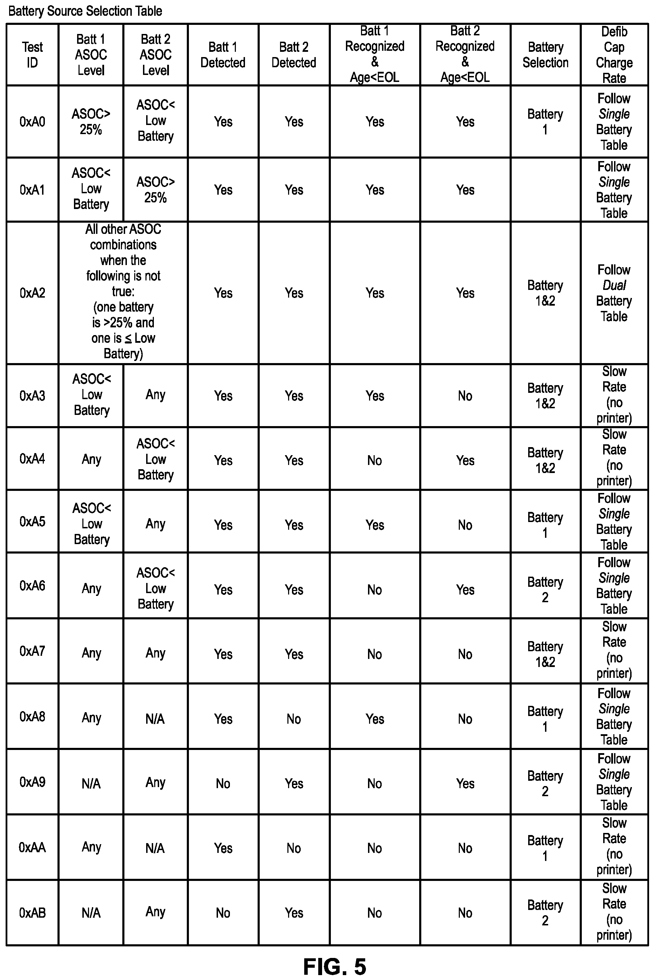

The processing unit 350 may also be configured to select the first battery unit 390 or the second battery unit 392 to provide power for the operation the medical device 302. The processing unit 350 may be configured to monitor and evaluate the parameters and/or conditions of the battery units 390 and 392 to determine whether to select the first battery unit 390 or the second battery unit 392 to provide power for the operation of the medical device 302. The criteria used by the processing unit 350 to select the first battery under 390 and/or the second battery unit 392 to provide power is set forth in the table of Figure 5. It will be recognized that the medical device 302 may use any suitable criteria for selecting the first battery unit 392 and/or the second battery unit 392 to provide power for the operation of the medical device 302. The values of the table set forth in Figure 5 may be stored in the memory unit 352 of the medical device 302 and used by the processing unit 350 as a look-up table.

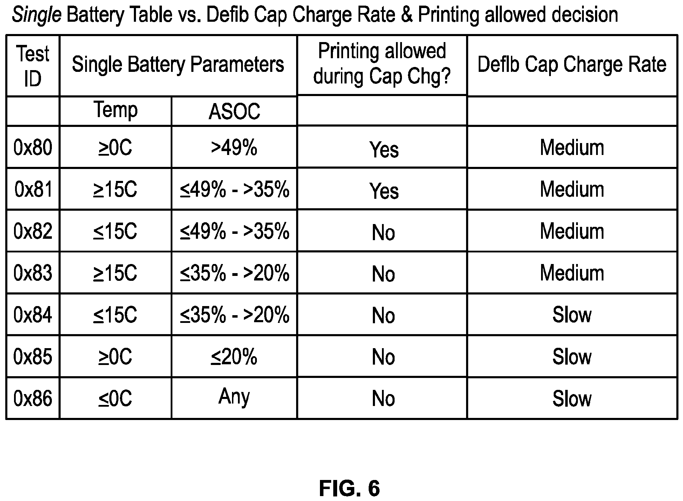

In some implementations, the processing unit 350 may select the first battery unit 390 to provide power for the operation of the medical device 304 when the ASOC of the first battery unit 390 is greater than 25% and the ASOC of the second battery unit 392 is less than a low battery threshold (e.g., a preset value). Similarly, when the ASOC of the second battery unit 392 is greater than 25% and the ASOC of the first battery unit 390 is less than a low battery threshold (e.g., a preset value), the processing unit 350 may select the second battery unit 392 to provide power for the operation of the medical device 302. When the processing unit 350 selects the first battery unit 390 or the second battery unit 392 to provide power for the medical device 302, the charging module 360 may charge the energy storage module 362 based the table set forth in Figure 6 as further described below. It will be recognized that the medical device 302 may use any suitable criteria for selecting the first battery unit 392 and the second battery unit 392 to provide power for the operation of the medical device 302.

The processing unit 350 of the medical device 302 may also be configured to select both of the first and second battery units 390 and 392 to provide power for the operation of the medical device 302. The processing unit 350 may select both the first battery unit 390 and the second battery unit 392 based on the parameters and/or conditions of the battery units. The criteria used by the processing unit 350 to select the first and second battery units 390 and 392 to provide power for the medical device 302 may set forth in the table of Figure 5. It will be recognized that the medical device 302 may use any suitable criteria for selecting the first and second battery units 390 and 392 to provide power for the operation of the medical device 304.

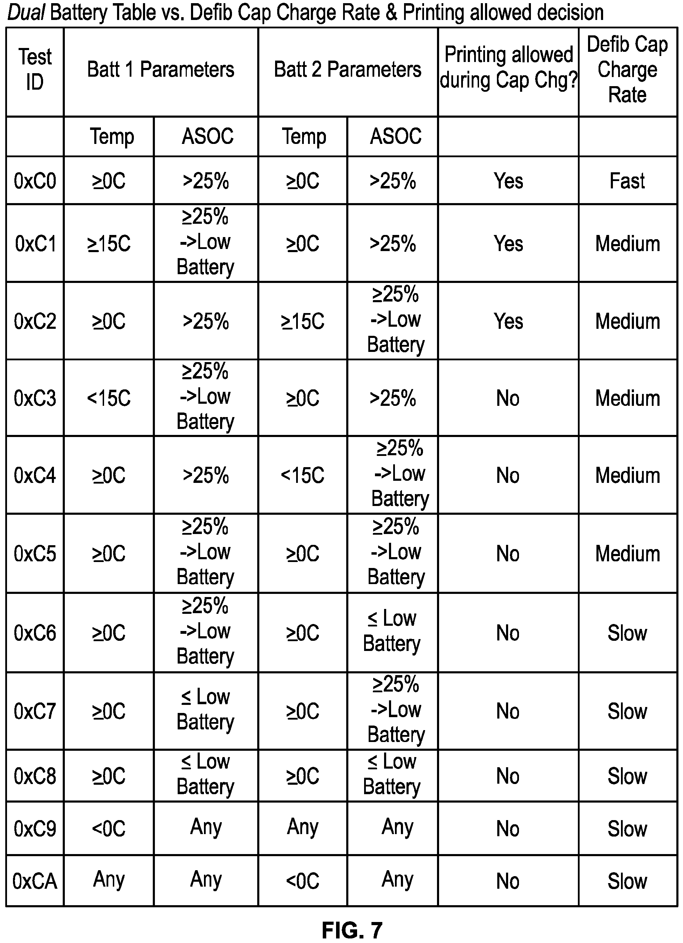

In some implementations, the processing unit 350 may select the first and second battery units 390 and 392 to provide power for the operation of the medical device 302 when the ASOC of one of the first or second battery units 390 and 392 is not greater than 25% and the ASOC of one of the first and second battery units 390 and 392 is not less than or equal to a low battery threshold (e.g., a preset threshold). When the processing unit 350 selects both of the first and second battery units 390 and 392 to provide power, the charging module 360 may charge the energy storage module 362 based on the table set forth in Figure 7.

Referring again to Figure 3, the charging module 360 of the medical device 302 may be configured to charge the energy storage module 362 before a defibrillation shock is delivered to a person. The charging module 360 may include charging circuitry for charging the energy storage module 362 to a selected voltage level that is determined based on a selected energy level for the defibrillation shock. During operation of the medical device 302, the charging module 360 may draw power from the first and/or second battery units 390 and 392 to charge the energy storage module 362. The first battery unit 390 and the second battery unit 392 may provide power equally to the charging module for charging the energy storage module 362 of the medical device 302. In some implementations, the charging circuit 130 may draw power from the first battery unit 390 and the second battery unit 392 at rates proportional to their respective charges for charging the energy storage module 362. For example, the battery unit having a higher remaining charge may provide greater than 50% of the power and the battery unit having the lower remaining charge may provide less than 50% of the power. However, when the remaining charge of the first battery unit 390 is above a first threshold value and the remaining charge of the second battery unit 392 is below a second threshold value, only the first battery unit 390 may provide power for charging the energy storage module 362. Similarly, when the remaining charge of the first battery unit 390 is below the first threshold value and the remaining charge of the second battery unit 392 is above the second threshold value, only the second battery unit may provide power for charging the energy storage module 362.

The charging module 360 may charge the energy storage module 362 at various rates, such as a maximum rate, a medium rate, and a slow rate. The processing unit 350 may change or alter the charging rate of the energy storage module 362 based on the conditions and/or parameters of the first and/or second battery units 390 and 392. The processing unit 350 may use the table set forth in Figure 6 to select a rate for the charging module 360 to charge the energy storage module 362 when the first battery unit 390 or the second battery units 392 provides power for the operation of the medical device 304. The values set forth in the table of Figure 6 may be stored in the memory unit 353 of the medical device 302 and used by the processing unit 350 as a look-up table.

In some implementations, the processing unit 350 may alter the charging rate of the energy storage module 362 based on a battery temperature and an ASOC of a battery unit providing power for the medical device 302. For example, the processing unit 350 may determine whether to adjust or change the rate for charging the energy storage module 362 based on a comparison of the ASOC of a battery unit to an ASOC threshold and the temperature of the battery unit to a temperature threshold. The processing unit 350 may use the table of Figure 6 to select a rate for the charging module 360 to charge the energy storage module 362. For example, when the ASOC of the battery unit providing power satisfies the ASOC threshold (e.g., the ASOC of the battery unit is equal to and/or above first ASOC threshold) and the temperature of the battery unit satisfies the temperature threshold (e.g., the temperature of the battery unit is equal to and/or above the first temperature threshold), the processing unit 350 may be configured to alter or change the rate at which the charging module 360 charges the energy storage module 362 according to the charging rates set forth in the table of Figure 6. In some examples, when the temperature of the battery unit providing power is equal to or exceeds 0 degrees Celsius and the ASOC of the buttery unit is greater that 49 percent, the processing unit 350 may be configured to select a medium charging rate at which the charging module 360 charges the energy storage module 362. When both the first and second battery units 390 and 392 are providing power for the operation of the medical device 304, the processing unit 350 may also be configured to alter or change the rate at which the charging module 360 charges the energy storage module 362 based on the battery parameters set forth in the table of Figure 7.

The processing unit may also be configured to implement algorithms (e.g., a back stop algorithm) to help manage the load on the medical device 302 under transient situations and/or high load conditions. For example, the processing unit 350 may change or alter the charging rate of the energy storage module 362 based on the conditions and/or parameters of the first and/or second battery units 390 and 392. In some implementations, the processing unit 350 may adjust the rate for charging the energy storage module 362 based on a comparison of a voltage of one or more of the battery units to a voltage threshold (e.g., 11.8 volts). When the medical device 302 unit is actively charging the energy storage module 362 and when a battery voltage of one of the batter units drops below or satisfies a voltage threshold (e.g., 11.8 volts), the processing unit 350 may change the rate at which the charging module 360 charges the energy storage device. The charging rate of the energy storage module 362 may be changed by adjusting the duty cycle of an output signal (e.g., a pulse width modulated (PWM) output signal) of the processing unit 350.

The rate at which the charging module 360 charges the energy storage module 362 may be changed from a first or maximum rate to a medium or slow rate by adjusting the duty cycle of the PWM output signal of the processing unit 350. In some implementations, the processing unit 350 may select or set the duty cycle of the PWM output signal equal to 20% for a fast to medium charging rate, the duty cycle of the PWM output signal equal to 60% for a fast to slow charging rate, the duty cycle of the PWM output signal equal to 40% for a medium to slow charge rate, and the duty cycle of the PWM output signal equal to 30% for slow to slower charge rate.

Further, the processing unit 350 may alter or change the charging rate of the energy storage module 362 of the medical device 302 based on a system or current load of the first battery unit 390 and/or the second battery unit 392. The processing unit 350 may adjust (e.g. slow down) the rate for charging the energy storage module 362 based on a comparison of the current load of one or more of the battery units to a current load threshold. For example, when the current load of one of the battery units satisfies a current load threshold (e.g., the current load of a battery unit is equal to and/or exceeds the current load threshold), the processing unit 350 may change the rate at which the charging module 360 charges the energy storage device. In some implementations, the processing unit 350 may take two consecutive samples within a 1.5-second window and determine whether the current load equals and/or exceeds a current load threshold (e.g., 9 Amps). In other implementations, the processing unit 350 may take four consecutive samples within a 3-second window and determine whether the current load equals and/or exceeds a current load threshold (e.g., 8.3 Amps). The charging rate of the energy storage module 362 may be changed by adjusting the duty cycle of the PWM output signal of the processing unit 350.

The rate at which the charging module 360 charges the energy storage module 362 may be changed from a first or maximum rate to a medium or slow rate by adjusting the duty cycle of the PWM output signal of the processing unit 350. In some implementations, the processing unit 350 may select or set the duty cycle of the PWM output signal equal to 20% for a fast to medium charging rate, the duty cycle of the PWM output signal equal to 60% for a fast to slow charge rate, the duty cycle of the PWM output signal equal to 40% for a medium to slow charge rate, the duty cycle of the PWM output signal setting equal to 30% for a slow to slower charge rate. As a result, the processing unit 350 may mitigate situations when a current load (e.g., on a bus) may exceed a threshold and is not detected before the initiation of the charging of the energy storage module 362, then the processing unit 350 may adjust or slowdown the charging rate by selecting one of the duty cycles for the PWM output signal of the processing unit 350 described above.

The energy storage module 362 of the medical device 302 may store electrical energy in the form of an electrical charge for delivery of a defibrillation pulse or shock to a person being treated for a medical condition, such as a cardiac arrest. The energy storage module 362 may be charged by the charging module 360 to a desired energy level (e.g., between 50 Joules to 360), as controlled by processing unit 350. For instance, for an adult, the processing unit 350 may select an energy level from an adult energy sequence that includes energy levels of 200 Joules, 300 Joules, and 360 Joules. For a pediatric patient, the processing unit 350 may select an energy level from a pediatric energy sequence that includes energy levels of 50 Joules, 75 Joules, and 90 Joules. The energy storage module 362 may include one or more capacitors that stores the energy to be delivered to a person as a defibrillation shock. When the energy of the energy storage module 362 reaches the desired energy level, the defibrillation shock may be delivered manually or automatically. For example, the user interface 354 may provide an indication to the user that medical device 302 is ready to deliver a defibrillation pulse or shock.

The discharge circuit 364 of the medical device may enable the energy stored in the energy storage module 362 to be discharged to a person. The electrical energy may be discharged at a selected energy level, via the ports or nodes 376 and 378, to the defibrillation electrodes 372 and 374 to cause a shock to be delivered to the person. The discharge circuit 364 can be controlled by the processing unit 350 or directly by the user via the user interface 354. In some implementations, the discharge circuit 364 can include one or more switches that, when activated, couple the energy storage module 362 to the ports or nodes 376 and 378 to deliver a defibrillation shock to person via defibrillation electrodes 372 and 374. For example, the processing unit 350 may activate the switches to electrically connect the energy storage module 362 to the defibrillation electrodes 372 and 374, and thereby deliver the defibrillation shock to person. The switches can be made in a number of ways and may be formed, for example, of electrically operated relays. Alternatively, the switches may comprise an arrangement of solid-state devices such as silicon-controlled rectifiers or insulated gate bipolar transistors.

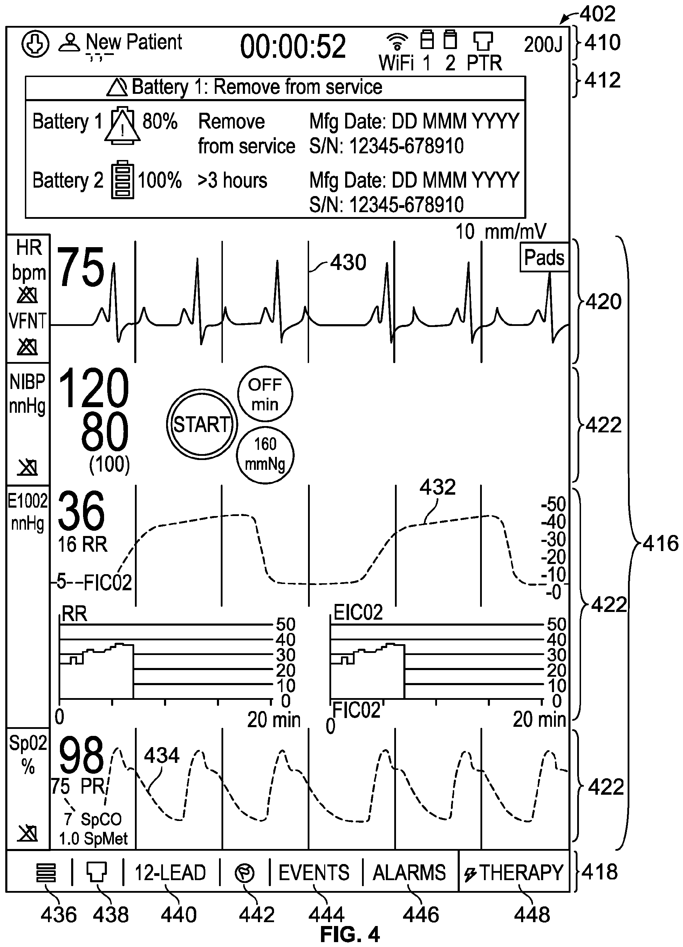

Figure 4 illustrates a GUI 402, in accordance with an example implementation. The GUI 402 may be displayed by a medical device, such as the medical device of Figures 1 and 2. For example, a medical device may be configured to generate a display of or visually present the GUI 402 on a touchscreen to enable a user (e.g., a healthcare professional) to interact with the medical device through user-selectable on-screen graphical items or components (e.g., buttons, menus, widgets, scroll bars, graphical objects, audio indicators, icons, etc.). The user-selectable user-interface items may convey information and represent actions that may be taken by a user. The user may select the user-selectable user-interface items by pressing or selecting areas on the touchscreen displaying the items. These user-selectable user-interface items may be enhanced with sounds, or visual effects like change in color, transparency, or drop shadows to facilitate interaction with the GUI 402.

As shown in Figure 4, the GUI 402 includes a status bar 410, a messaging area 412, a content area 416, and a navigation task bar 418. The messaging area 412 of the GUI 410 may display messages about the battery units (e.g., the first battery unit 390 and/or the second battery unit 392) of a medical device. As shown in Figure 4, the GUI 402 may display information about the end of life status of a first battery unit and/or a second battery unit. As shown, the GUI 402 indicates that the first battery unit should be removed from service. The GUI 402 may also provide additional information about the battery units of the medical device, such as the battery charge levels (e.g., remaining charge), battery replacement or removal indicators, manufacturing dates, serial numbers, etc. Additionally, the GUI 410 may provide audible alarms or warnings when a battery unit of the medical device has reached its end of life and/or should be removed and replaced.

The content area 416 of the GUI 402 may show patient data including physiologic parameters and waveforms output or displayed by the medical device as well as provided by physiologic sensors. The content area 416 may include multiple channels, including a primary channel 420 for displaying a primary waveform 430 and multiple secondary channels 422 for displaying secondary data, such as a secondary waveform 432. In Figure 4, the primary waveform 430 is shown as an ECG waveform, obtained using defibrillation electrodes or sensors. In some examples, the secondary channels 422 may display one or more ECG waveforms as well as waveforms indicative of other physiological parameters or data (e.g., a plethysmograph, a capnography waveform, etc.). The secondary channel 422 may also display numerical values, such as a heart rate, respiration rate, a body temperature, and any other physiological parameters. As illustrated, the primary channel 420 is positioned above the secondary channels 422. Although multiple secondary channels 422 are shown, in other examples, a GUI may only display a single secondary channel.

The GUI 402 may also be configured to display waveforms next to a side rectangle having a particular color and labelled by a physiologic parameter to which the waveform pertains. For example, the GUI includes the waveform 430 for heart rate (HR), the waveform 432 for End-tidal CO2 (EtCO2), which indicates the partial pressure or maximal concentration of carbon dioxide (CO2) at the end of an exhaled breath, and the waveform 434 for SpO2. The GUI 402 may also display NIBP values for a patient or person being monitored and/or treated. Further, the GUI 402 may display a navigation task bar 418 at the bottom of the GUI 402. The navigation task bar 418 may have various tabs and menu options. As shown, the navigation task bar 418 includes a menu button 436, a print button 438, a 12-Lead button 440, a generic event button 442, an events button 444, an alarms button 446, and therapy button 448.

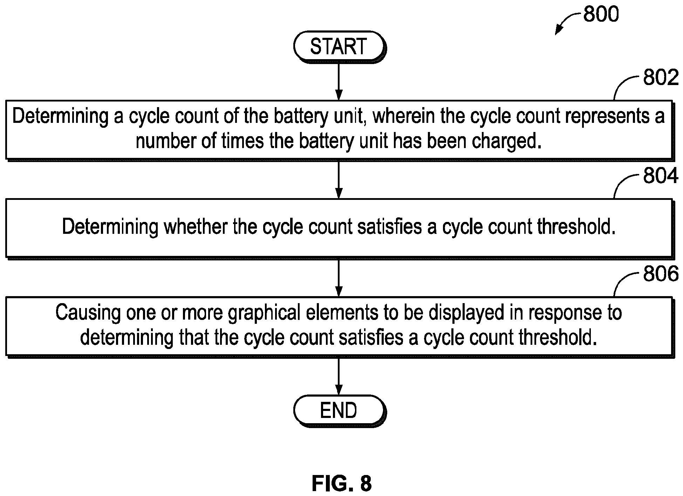

Figure 8 illustrates a flow chart of a method 800 for detecting the end-of-life a battery unit of a medical device, according to example implementations. The method 800 represents an example method that may include one or more operations, functions, or actions, as depicted by one or more of blocks 802-806, each of which may be performed by any of the medical devices or systems described herein. For instance, medical device 202 depicted in Figure 2 may enable execution of method 800.

Those skilled in the art will understand that the flowchart of Figure 8 herein illustrates functionality and operations of certain implementations of the present disclosure. In this regard, each block of the flowchart may represent a module, a segment, or a portion of program code, which includes one or more instructions executable by one or more processors for implementing specific logical functions or steps in the process. The program code may be stored on any type of computer readable medium, for example, such as a storage device including a disk or hard drive.

In addition, each block may represent circuitry that is wired to perform the specific logical functions in the process. Alternative implementations are included within the scope of the example implementations of the present application in which functions may be executed out of order from that shown or discussed, including substantially concurrent or in reverse order, depending on the functionality involved, as would be understood by those reasonably skilled in the art.

At block 802, the method 800 involves determining a cycle count of the battery unit, wherein the cycle count represents a number of times the battery unit has been charged. A medical device may be operated to monitor and provide treatment or care to a person or patient experiencing a medical condition, such as a cardiac arrest or any other cardiac rhythm abnormality. The medical device may utilize one or more battery units to provide power thereto. The medical device may determine the end of the life status of a battery unit by monitoring the number of charging cycles of a battery unit and comparing a cycle count to a cycle count threshold.

At block 804, the method 800 involves determining whether the cycle count satisfies a cycle count threshold. The medical device may compare the cycle count of a battery unit to a cycle count threshold. The cycle count threshold may represent the number of times a battery unit has been charged. The medical device may determine whether the cycle count of a battery unit of the medical device satisfies a cycle count threshold (e.g., the cycle count is equal to and/or above the cycle count threshold).

At block 804, the method 800 involves causing one or more graphical elements to be displayed in response to determining that the cycle count satisfies a cycle count threshold. When the cycle count of a battery unit of the medical device satisfies the cycle count threshold (e.g., the cycle count is equal to and/or above the cycle count threshold), the medical device may be configured to cause information (e.g., graphical representations) to be provided or displayed indicating the first battery unit is at its end of life and needs to be replaced.

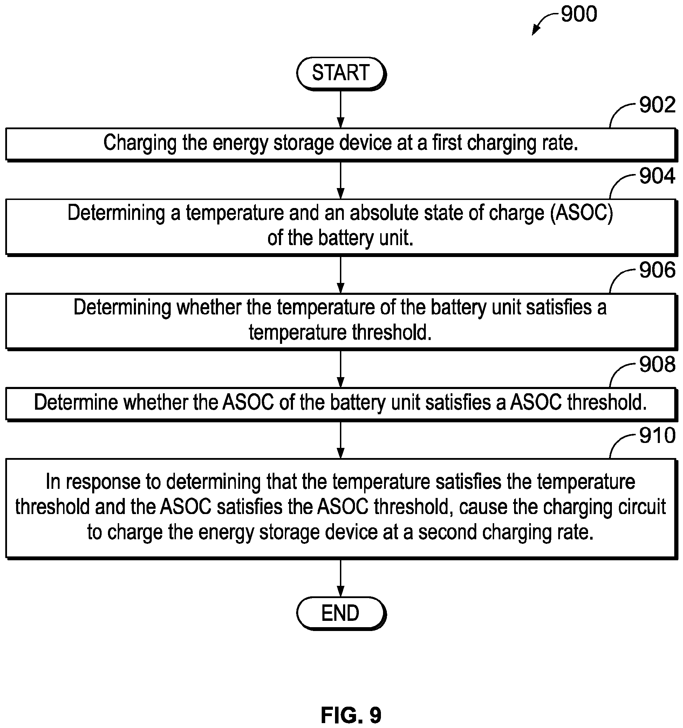

Figure 9 illustrates a flow chart of a method 900 of charging an energy storage device of a portable medical device, according to example implementations. The method 900 represents an example method that may include one or more operations, functions, or actions, as depicted by one or more of blocks 902-910, each of which may be performed by any of the medical devices or systems described herein. For instance, the medical device 302 depicted in Figure 2 may enable execution of method 900.

Those skilled in the art will understand that the flowchart of Figure 9 illustrates functionality and operations of certain implementations of the present disclosure. In this regard, each block of the flowchart may represent a module, a segment, or a portion of program code, which includes one or more instructions executable by one or more processors for implementing specific logical functions or steps in the process. The program code may be stored on any type of computer readable medium, for example, such as a storage device including a disk or hard drive.

In addition, each block may represent circuitry that is wired to perform the specific logical functions in the process. Alternative implementations are included within the scope of the example implementations of the present application in which functions may be executed out of order from that shown or discussed, including substantially concurrent or in reverse order, depending on the functionality involved, as would be understood by those reasonably skilled in the art.

At block 902, the method 900 involves charging the energy storage device of the portable medical device at a first charging rate. A medical device may be operated to monitor and provide treatment or care to a person or patient experiencing a medical condition, such as a cardiac arrest or any other cardiac rhythm abnormality. The medical device may include an energy storage module to store electrical energy in the form of an electrical charge for delivery of a defibrillation pulse or shock to the person being treated for a medical condition. The energy storage module can be charged by a charging module to a desired energy level. The charging module may charge the energy storage module at various rates, such as a maximum rate, a medium rate, and a slow rate.

At block 904, the method involves determining a temperature and an absolute state of charge (ASOC) of the battery unit. The medical device may determine the temperature and the ASCO of a battery unit of the medical device. At block 906, the method involves determining whether the temperature of the battery unit satisfies a temperature threshold. The medical device may compare the temperature of a battery unit to a temperature threshold and may determine whether the temperature of the battery unit is equal to and/or above the temperature threshold. At block 908, the method involves determining whether the ASOC of the battery unit satisfies an ASOC threshold. The medical device may compare the ACOC of the battery unit to an ASOC threshold and determine whether the ASCO level of the battery unit is equal to and/or above the ASCO threshold.

At block 910, the method involves, in response to determining that the temperature satisfies the temperature threshold and the ASOC satisfies the ASOC threshold, causing the charging circuit to charge the energy storage device at a second charging rate. The medical device may change or alter the charging rate of the energy storage module based on the conditions and/or parameters of a battery unit. For example, the medical device may determine an ASOC and a temperature of the battery unit and may alter the charging rate of the energy storage module based on the ASOC and the temperature of the battery unit. For example, when the medical device determines that the ASCO and the temperature of the battery unit are equal to and/or above the ASCO threshold and temperature threshold, respectively, the medical device may be configured to alter or change the rate at which the charging module charges the energy storage device.

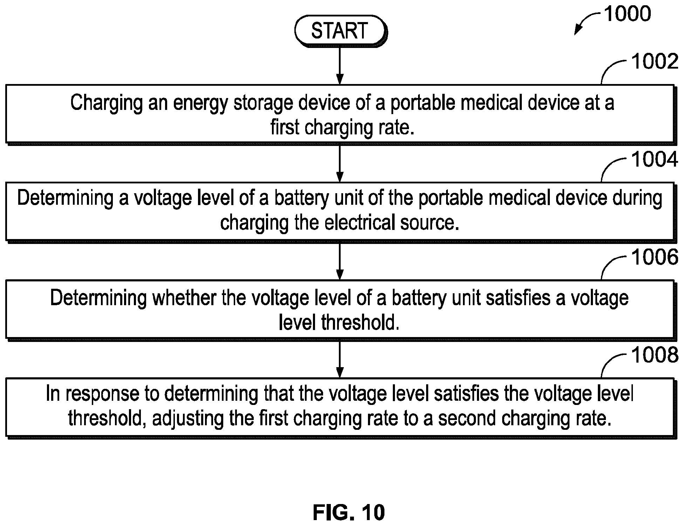

Figure 10 illustrates a flow chart of a method 1000 of charging an energy storage device of a portable medical device, according to example implementations. The method 1000 represents an example method that may include one or more operations, functions, or actions, as depicted by one or more of blocks 1002-1008, each of which may be performed by any of the medical devices or systems described herein. For instance, the medical device 202 depicted in Figure 2 may enable execution of method 1000.

Those skilled in the art will understand that the flowchart of Figure 10 illustrates functionality and operations of certain implementations of the present disclosure. In this regard, each block of the flowchart may represent a module, a segment, or a portion of program code, which includes one or more instructions executable by one or more processors for implementing specific logical functions or steps in the process. The program code may be stored on any type of computer readable medium, for example, such as a storage device including a disk or hard drive.

In addition, each block may represent circuitry that is wired to perform the specific logical functions in the process. Alternative implementations are included within the scope of the example implementations of the present application in which functions may be executed out of order from that shown or discussed, including substantially concurrent or in reverse order, depending on the functionality involved, as would be understood by those reasonably skilled in the art.

At block 1002, the method 1000 involves charging the energy storage device of a portable medical device at a first charging rate. A medical device may be operated to monitor and provide treatment or care to a person or patient experiencing a medical condition, such as a cardiac arrest or any other cardiac rhythm abnormality. The medical device may include an energy storage module to store electrical energy in the form of an electrical charge for delivery of a defibrillation pulse or shock to a person being treated for a medical condition. The energy storage module may be charged by a charging module to a desired energy level. The charging module may charge the energy storage module at various rates, such as a maximum rate, a medium rate, and a slow rate.

At block 1004, the method 1000 involves determining a voltage level of a battery unit of the portable medical device during charging of the energy storage device. The medical device may change or alter the charging rate of the energy storage module based on the conditions and/or parameters of a battery unit. For example, the medical device may determine a voltage level of the battery unit and may alter the charging rate of the energy storage module based on the voltage of the battery unit. At block 1006, the method 1000 involves determining whether the voltage level of a battery unit satisfies a voltage level threshold. The medical device may compare the voltage of the battery unit to a voltage threshold and may determine whether the battery voltage is less than or equal to the voltage threshold.

At block 1008, the method 1000 involves, in response to determining that the voltage level satisfies the voltage level threshold, adjusting the first charging rate to a second charging rate. When the voltage of the battery unit of the medical device satisfies the voltage threshold (e.g., the voltage of the battery unit is equal to and/or above the voltage threshold), the medical device may be configured to alter or change the rate at which the charging module charges the energy storage device.

The description of the different advantageous arrangements has been presented for purposes of illustration and description, and is not intended to be exhaustive or limited to the examples in the form disclosed. Many modifications and variations will be apparent to those of ordinary skill in the art. Further, different advantageous examples describe different advantages as compared to other advantageous examples. The example or examples selected are chosen and described in order to best explain the principles of the examples, the practical application, and to enable others of ordinary skill in the art to understand the disclosure for various examples with various modifications as are suited to the particular use contemplated.

The embodiments described herein can be realized in hardware, software, or a combination of hardware and software. For example, the embodiments can be realized in a centralized fashion in at least one computer system or in a distributed fashion where different elements are spread across interconnected computer systems. Any kind of computer system or other apparatus adapted for carrying out the methods described herein can be employed. Further, the embodiments described herein can be embedded in a computer program product, which includes all the features enabling the implementation of the operations described herein and which, when loaded in a computer system, can carry out these operations.

The flowcharts and block diagrams described herein illustrate the architecture, functionality, and operation of possible implementations of systems, methods, and apparatus according to various illustrative embodiments. In this regard, each block in the flowcharts or block diagrams may represent a module, segment, or portion of code, which comprises one or more executable instructions for implementing the specified logical function or functions. It should also be noted that, in some alternative implementations, the functions noted in a block may occur out of the order noted in the drawings. For example, the functions of two blocks shown in succession may be executed substantially concurrently, or the functions of the blocks may sometimes be executed in the reverse order, depending upon the functionality involved.

Additionally, instances in this specification where one element is "coupled" to another element can include direct and indirect coupling. Direct coupling can be defined as one element coupled to and in some contact with another element. Indirect coupling can be defined as coupling between two elements not in direct contact with each other, but having one or more additional elements between the coupled elements. Further, as used herein, securing one element to another element can include direct securing and indirect securing. Additionally, as used herein, "adjacent" does not necessarily denote contact. For example, one element can be adjacent another element without being in contact with that element.

As used herein, a system, apparatus, structure, article, element, component, or hardware "configured to" perform a specified function is indeed capable of performing the specified function without any alteration, rather than merely having potential to perform the specified function after further modification. In other words, the system, apparatus, structure, article, element, component, or hardware "configured to" perform a specified function is specifically selected, created, implemented, utilized, programmed, and/or designed for the purpose of performing the specified function. As used herein, "configured to" denotes existing characteristics of a system, apparatus, structure, article, element, component, or hardware which enable the system, apparatus, structure, article, element, component, or hardware to perform the specified function without further modification. For purposes of this disclosure, a system, apparatus, structure, article, element, component, or hardware described as being "configured to" perform a particular function may additionally or alternatively be described as being "adapted to" and/or as being "operative to" perform that function.

By the term "substantially" and "about" used herein, it is meant that the recited characteristic, parameter, or value need not be achieved exactly, but that deviations or variations, including for example, tolerances, measurement error, measurement accuracy limitations and other factors known to skill in the art, may occur in amounts that do not preclude the effect the characteristic was intended to provide.

Unless otherwise indicated, the terms "first," "second," etc. are used herein merely as labels, and are not intended to impose ordinal, positional, or hierarchical requirements on the items to which these terms refer. Moreover, reference to, e.g., a "second" item does not require or preclude the existence of, e.g., a "first" or lower-numbered item, and/or, e.g., a "third" or higher-numbered item.

While apparatus has been described with reference to certain examples, it will be understood by those skilled in the art that various changes can be made and equivalents can be substituted without departing from the scope of the claims. Therefore, it is intended that the present apparatus not be limited to the particular examples disclosed, but that the disclosed apparatus include all embodiments falling within the scope of the appended claims.

## Claims

1. A portable medical device comprising:
a display;
a defibrillator port configured to enable a high energy output;
a battery unit; and
a processor configured to:
determine a charge level of the battery unit;
determine, when the charge level of the battery unit satisfies a charge level threshold, a temperature of the battery unit and an absolute state of charge (ASOC) of the battery unit;
determine whether the temperature of the battery unit satisfies a temperature threshold;
determine whether the ASOC of the battery unit satisfies a ASOC threshold; and
at least in response to determining that the temperature satisfies the temperature threshold and the ASOC satisfies the ASOC threshold, cause one or more graphical elements to be displayed on the display.

2. The portable medical device of claim 1, wherein the processor is further configured to:
determine a cycle count of the battery unit, wherein the cycle count represents a number of times the battery unit has been charged;
determine whether the cycle count satisfies a cycle count threshold; and
in response to determining that the cycle count satisfies the cycle count threshold, cause the one or more graphical elements to be displayed on the display.

3. The portable medical device of claim 1, wherein the one or more graphical elements includes an indicator configured to indicate that the battery unit needs to be replaced, or
the device optionally further comprising a housing surrounding the battery unit, wherein the portable medical device comprises a defibrillator assembly or a patient monitoring device, wherein the battery unit comprises a lithium battery or an alkaline battery, and wherein the battery unit is detachable from the portable medical device, or
wherein optionally the ASOC threshold is 75 percent, and wherein the temperature threshold is 15 degrees Celsius.

4. The portable medical device of claim 2, wherein the cycle count threshold is 300.

5. The portable medical device of claim 2, wherein the battery unit comprises a first battery unit, wherein the cycle count comprises a first cycle count, and further comprising a second battery unit, wherein the processor is further configured to:
determine a second cycle count of the second battery unit, wherein the second cycle count represents a number of times the second battery unit has been charged;
determine whether the second cycle count satisfies the cycle count threshold; and
in response to determining that the second cycle count of the battery unit satisfies the cycle count threshold, cause one or more additional graphical elements to be displayed on the display.

6. The portable medical device of claim 1, wherein the battery unit comprises a first battery unit, and further comprising a second battery unit, wherein the processor is further configured to:
determine a second charge level of the second battery unit;
determine, when the second charge level of the second battery unit satisfies the charge level threshold, a second temperature of the second battery unit and a second ASOC of the second battery unit;
determine whether the second temperature of the second battery unit satisfies the temperature threshold;
determine whether the second ASOC of the second battery unit satisfies the ASOC threshold; and
in response to determining that the second temperature satisfies the temperature threshold and the second ASOC satisfies the ASOC threshold, cause one or more additional graphical elements to be displayed on the display.

7. A method of monitoring a battery unit of a medical device, the method comprising:
determining, by one or more processors, a charge level of the battery unit;
determining, when the charge level of the battery unit satisfies a charge level threshold, a temperature of the battery unit and an absolute state of charge (ASOC) of the battery unit;
determining whether the temperature of the battery unit satisfies a temperature threshold;
determining whether the ASOC of the battery unit satisfies a ASOC threshold; and
at least in response to determining that the temperature satisfies the temperature threshold and the ASOC satisfies the ASOC threshold, causing one or more graphical elements to be displayed on the display.

8. The method of claim 7, further comprising:
determining a cycle count of the battery unit, wherein the cycle count represents a number of times the battery unit has been charged;
determining whether the cycle count satisfies a cycle count threshold; and
in response to determining that the cycle count satisfies the cycle count threshold, cause the one or more graphical elements to be displayed on the display.

9. The method of claim 7, wherein the one or more graphical elements includes an indicator configured to indicate that the battery unit needs to be replaced, or
wherein optionally the ASOC threshold is 75 percent, and wherein the temperature threshold is 15 degrees Celsius, or
wherein optionally the battery unit comprises a lithium battery or an alkaline battery.

10. The method of claim 7, wherein the battery unit comprises a first battery unit, wherein the medical device comprises a second battery unit, and wherein the method further comprises:
determining a second charge level of the second battery unit;
determining, when the second charge level of the second battery unit satisfies the charge level threshold, a second temperature of the second battery unit and a second ASOC of the second battery unit;
determining whether the second temperature of the second battery unit satisfies the temperature threshold;
determining whether the second ASOC of the second battery unit satisfies the ASOC threshold; and
in response to determining that the second temperature satisfies the temperature threshold and the second ASOC satisfies the ASOC threshold, causing one or more additional graphical elements to be displayed on the display.

11. A non-transitory computer-readable medium comprising instructions that, when executed by one or more processors, cause the one or more processors to perform operations for monitoring a battery unit of a medical device, the operations comprising:
determining a charge level of the battery unit;
determining, when the charge level of the battery unit satisfies a charge level threshold, a temperature of the battery unit and an absolute state of charge (ASOC) of the battery unit;
determining whether the temperature of the battery unit satisfies a temperature threshold;
determining whether the ASOC of the battery unit satisfies a ASOC threshold; and
at least in response to determining that the temperature satisfies the temperature threshold and the ASOC satisfies the ASOC threshold, causing one or more graphical elements to be displayed on the display.

12. The non-transitory computer-readable medium of claim 11, wherein the operations further comprise:
determining a cycle count of the battery unit, wherein the cycle count represents a number of times the battery unit has been charged;
determining whether the cycle count satisfies a cycle count threshold; and
in response to determining that the cycle count satisfies the cycle count threshold, cause the one or more graphical elements to be displayed on the display.

13. The non-transitory computer-readable medium of claim 11, wherein the one or more graphical elements includes an indicator configured to indicate that the battery unit needs to be replaced, or
wherein optionally the ASOC threshold is 75 percent, and wherein the temperature threshold is 15 degrees Celsius.

14. The non-transitory computer-readable medium of claim 11, wherein the battery unit comprises a first battery unit, wherein the medical device comprises a second battery unit, and wherein the operations further comprise:
determining a second charge level of the second battery unit;
determining, when the second charge level of the second battery unit satisfies the charge level threshold, a second temperature of the second battery unit and a second ASOC of the second battery unit;
determining whether the second temperature of the second battery unit satisfies the temperature threshold;
determining whether the second ASOC of the second battery unit satisfies the ASOC threshold; and
in response to determining that the second temperature satisfies the temperature threshold and the second ASOC satisfies the ASOC threshold, causing one or more additional graphical elements to be displayed on the display.

15. The non-transitory computer-readable medium of claim 11, wherein the battery unit comprises a lithium battery or an alkaline battery.
